# EUROPEAN PATENT SPECIFICATION

(11) **EP 0 378 132 B1**
(45) Date of publication and mention of the grant of the patent: **27.11.1996**
(21) Application number: 90100236.0
(22) Date of filing: 05.01.1990
(51) Int. Cl.: A61N 1/30

(54) **A device for the administration of medication by iontopheresis for local - regional treatment.**
Vorrichtung zum Eingeben von Medikamenten durch Iontophorese für örtliche oder regionale Behandlungen
Dispositif d'administration de médicaments par ionophorèse pour un traitement local ou régional

(30) Priority: 09.01.1989 ES 8900058
(43) Date of publication of application: 18.07.1990
(73) Proprietor: CIT IONOFOR, S.L., Palma de Mallorca (Baleares) (ES)
(72) Inventor: Tomas Justribo, José Ramon, 08034 Barcelona (ES)
(74) Representative: Kador & Partner

(56) References cited:
- WO-A-86/07268
- WO-A-86/07269
- WO-A-88/08729
- US-A- 4 116 238
- US-A- 4 624 661

## Description

At present, as it is known the administration of pharmaceutical products of an active nature to both humans and animals is based on the dispensing of these active products into the most appropriate part of the patient's circulatory system whether this be by oral ingestion, by various means of injection or by insertion in the rectum, etc.

In US-A-4,116,238 there is disclosed a regulated current high voltage source for use in driving an iontophoresis probe, in which the effect of clogging of pipettes and other phenomenon can be counteracted by the capability of the source to provide instantaneously, upon demand, extremely high voltage outputs. The source employs a DC-to-DC inverter, comprising a blocking oscillator and pulse transformer, with the load provided in the output circuit of the transformer in a feedback arrangement to a regulating amplifier and driver circuit for the DC input to the pulse transformer and blocking oscillator.

In US-A-4,624,661 there is disclosed a drug dispensing system providing accurate drug dispensing to an individual in critical and non-critical medical care situations. This drug dispensing system is designed for dispensing drugs from a reservoir through a drug dispenser according to a preselected algorithm controlling a motor where a CPU pumps the drug. In this dispensing system, an external controller for the drug dispenser stores a plurality of parameters including a patient number, name, drug dispenser number, date, time, drug, and algorithms for dispensing the drug including dosage levels and frequency of dosage, where the algorithm is stored in the external controller and the program is subsequently stored in the CPU of the drug dispenser.

One frequently encounters problems in the administration of such medication as it is currently known in that the concentration of the active product is insufficiently high in the pathologically affected zone and any increase of the dose in some instances may be dangerous due to the possibility of producing secondary effects in other parts of the organism or even prove to the lethal.

It is the object of the invention to provide a device for applying a pharmaceutical substance that can be placed in a controlled manner at the required area of an organism.

This object is achieved by means of a device for applying a pharmacological substance, having the features of claim 1. The device optionally possesses the further advantageous features of claim 2 and/or claim 3.

Preferred embodiments of this device are subject to the subclaims.
The device for applying a pharmaceutical substance according to the invention has the effects that the overall doses as well as the concentration of the substance to be applied can be decreased with respect to devices according to the state of the art.

This device is designed to solve the above problem by providing a means of controlling the efficient and simple regulation of the pharmaceutically active concentrate in the specific parts of the human anatomy correctly targeted in such a way that excessive levels in other parts of the organism are avoided.

The equipment is applicable to oncology, arthritis and other types of acute or chronic illnesses or infections of a degenerative or other nature.

By way of example, the device can be applied to oncology with the capability of achieving a better cytostatic concentration in the precise locality of the neoplasm, thus obtaining a more effective application of the medication without the difficulties normally met using the usual applicational devices. It is well known that the therapeutic dosage of the cytostatic is at almost the same level as the lethal dosage so that dosage increases cannot be applied following initial satisfactory results, the state of the patient's health nor permitting such an increase.

The administration method used in devices used up to now have been the following: oral, intramuscular, intrathecal and finally intravenous by means of a venoclysis. Of all these the last is the most used although exercising a constant vigilance from the haematological point of view, arresting the medication once the haematology so indicates. Reasonably satisfactory results are thus obtained over a long period of time but nevertheless there remain focuses of neoplasm untreated by the cytostatic so a long term cure is not achieved. Recently the use of Lymphocytes T and K has been tried with truly astonishing results but even in these cases one cannot claim a cure. This treatment however is worthy of consideration given that the application of the device, object of this patent for the administration of cytostatics allows a simultaneous reinforcement of the immuno-therapeutic treatment, operating in this manner with two distinct methods simultaneously, aggregating the effects and with the same degree of risk for the patient during the disorders of the haemopoietic organs.

This equipment, in principle can be utilized in the case of topographically localized malignant neoplasm and the metastatis including those where the primary locality is not known but are a local regional spread, as well as in other illnesses acting in localized areas.

The object of this invention is based on the injection of a pharmacological substance and the simultaneous creation of an electric field capable of provoking mobile iontophoresis. Both operations must be closely controlled and the equipment must be able to carry them out simultaneously in various pairs of electrode - injection needles producing thus a spread of the substance in a star formation.

The apparatus, object of this invention consists essentially of an electric field generator module, a micro-injection pump module, the injection electrodes and a control processor.

The programmable voltage generator module or modules create an electric field, preferentially three dimensional, by means of one or several electrodes lodged appropriately within or on the patient to the treated. The micro injection pumps introduce one or several medications as programmed, through the electrode injectors, preferably simultaneously or on a dosification and the processor controls to a program the feed of the injected substance or substances to the required area, this by a control of the intensity and polarity of the electric field created by the electrodes and the regulation of the flow supplied by the injection pumps.

In more detail, the electric field generator comprises a number of identical sub-modules which are programmable as to polarity, voltage (between 0 and 60 volts), and current, the polarity being changeable at various time intervals. The equipment must meet the insulation requirements relevant to medical useage.

The sub-modules must be absolutely independent in order that spontaneously the polarity and voltage can be varied so as to provide a uniform distribution of the cytostatic substance.

The micro-injection pumps must be controllable from the control unit with a regulation of the flow and volume injected within the range of 0,1 to 5 cm3 per hour.

The electrode-injectors are in the form of a hypodermic needle, fully insulated over their entire body, the extremity only being live, and it is recommended that the platinum exterior be connected to earth.

The processor must be capable of controlling all the other components, the field generator, the flow and volume of the injected substance as well as the pattern of incidence governing the electric field which dictates the diffusion characteristics of the cytostatic substance in accordance with a program.

For a better understanding of the invention there are attached hereto, by way of example, drawings showing an explanation of the process and the device for administering pharmacological substances, the object of this patent.

Figure 1 shows in schematic form the iontophoresis equipment, object of this patent.

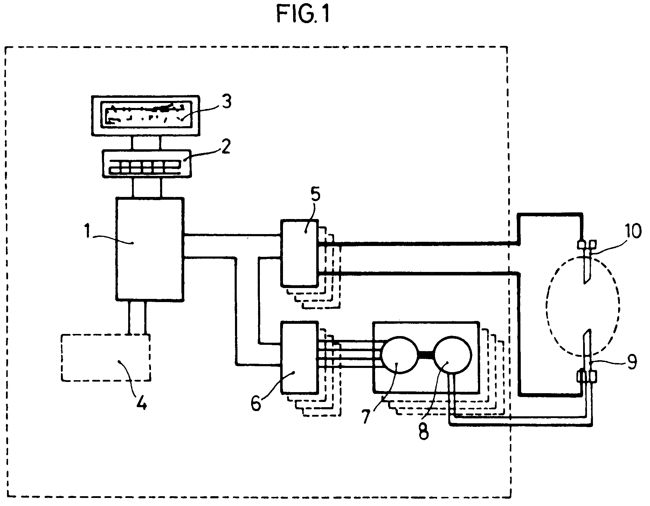

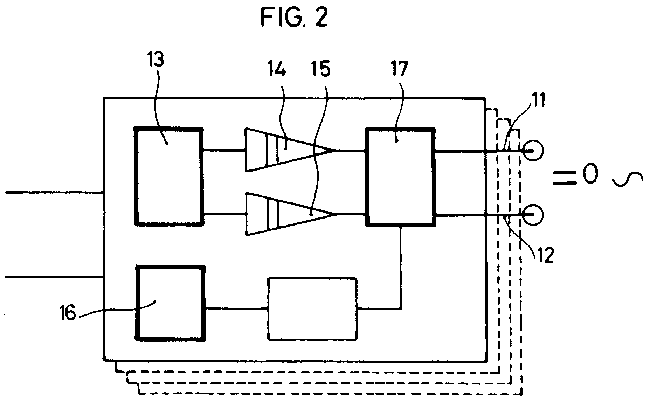

Figure 2 shows an explanatory sketch of the field generator.

Figure 3 shows a schematic longitudinal section of the active electrode for use with this invention.

As is shown in the above drawings this invention comprises essentially equipment made up of a processor 1 coupled up to a program entry system 2 for the entry of instructions and a display unit or screen 3. A RAM memory operates the data storage and electric field generator 5 feeds an active injector 9 and the electrode 10.

The injection pumps 7 and 8 are controlled by a separate control unit 6, the object of which is to feed the active injector 9.

The electric field generator 5 feeds the outgoing leads 11 and 12 and comprises an impulse stage or driver 13 and the isolator amplifiers 14 and 15. A feedback stage 16 supplies the current and impedance control unit 17.

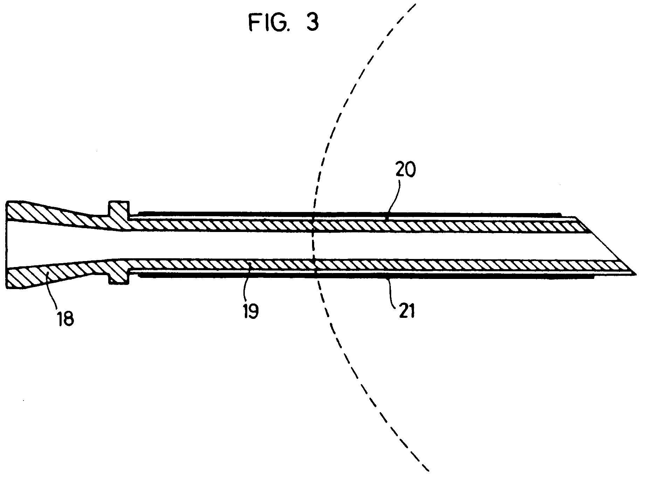

The injector shown in Figure 3 comprises an entry mouthpiece 18 extended in a tubular component 19 made from a material which is a good conductor of electricity, covered in a sleeve 20 in an insulating material and having an outer platinum covering 21. The active fluid passes through the interior of the tube 19.

## Claims

1. A device for applying a pharmacological substance, comprising at least one injection needle (9) for injecting the pharmacological substance, and a least one electrode (10), each needle (9) and electrode (10) being coupled to an electrical field generator (5) for creating a three-dimensional electrical field in the area of injection of the pharmacological substance thereby influencing its electrophoretic mobility, **characterized** in that the injection needle (9) is further coupled with a micro-injection pump (7,8), controlled by a control unit (6), for controlling the feed of the pharmacological substance into the needle (9), both the electrical field generator (5) and the control unit (6) being controlled by a programmable processor (1), to move the pharmacological substance in the required region by changing the polarity and intensity of the electrical field at different time intervals.

2. A device according to claim 1 which comprises a plurality of needles (9) and electrodes (10), and in which the electrical field generator (5) comprises a corresponding plurality of identical submodules.

3. A device according to claim 1 or claim 2 wherein each electrode (10) is in the form of a hypodermic needle formed of platinum and fully insulated over its entire body, only the extremity being live.

## Patentansprüche

1. Vorrichtung zur Anwendung einer pharmakologischen Substanz, die mindestens eine Injektionsnadel (9) zum Injizieren der pharmakologischen Substanz und mindestens eine Elektrode (10) umfaßt, wobei die Nadel (9) und die Elektrode (10) jeweils mit dem Generator (5) für ein elektrisches Feld verbunden sind, der im Injektionsbereich der pharmakologischen Substanz ein dreidimensionales elektrisches Feld erzeugt, wodurch deren elektrophoretische Beweglichkeit beeinflußt wird, dadurch gekennzeichnet, daß die Injektionsnadel (9) außerdem mit einer Mikroinjektionspumpe (7,8) gekoppelt ist, die von einem Regler (6) gesteuert wird, damit die Zufuhr der pharmakologischen Substanz in die Nadel (9) geregelt wird, wobei sowohl der Generator (5) für das elektrische Feld als auch der Regler (6) von einem programmierbaren Prozessor (1) gesteuert werden, wodurch die pharmakologische Substanz durch Änderung der Polarität und Intensität des elektrischen Feldes mit unterschiedlichen Zeitintervallen in den erforderlichen Bereich gebracht wird.

2. Vorrichtung nach Anspruch 1, die eine Anzahl Nadeln (9) und Elektroden (10) umfaßt und wobei der Generator (5) für das elektrische Feld eine entsprechende Anzahl identischer Submodule umfaßt.

3. Vorrichtung nach Anspruch 1 oder Anspruch 2, wobei jede Elektrode (10) in Form einer subkutanen Nadel vorliegt, die aus Platin ist und entlang des gesamten Nadelkörpers vollständig isoliert ist, wobei nur das Ende stromführend ist.

## Revendications

1. Dispositif pour appliquer une substance pharmacologique, comprenant au moins une aiguille d'injection (9) pour injecter la substance pharmacologique, et au moins une électrode (10), chaque aiguille (9) et électrode (10) étant couplé à un générateur (5) de champ électrique pour créer un champ électrique tridimensionnel dans la région d'une injection de la substance pharmacologique en iufluençant ainsi sa mobilité électrophorétique, caractérisé en ce que l'aiguille d'injection (9) est en outre couplée à une pompe à micro-injection (7, 8), commandée par un dispositif de commande (6), pour commander l'introduction de la substance pharmacologique dans l'aiguille (9), le générateur (5) de champ électrique et le dispositif de commande (6) étant tous deux commandés par un processeur programmable (1), de manière à déplacer la substance pharmacologique dans la région requise par modification de la polarité et de l'intensité du champ électrique à des intervalles de temps différents.

2. Dispositif selon la revendication 1, qui comprend une pluralité d'aiguilles (9) et d'électrodes (10), et dans lequel le générateur (5) de champ électrique comprend une pluralité correspondante de modules secondaires identiques.

3. Dispositif selon la revendication 1 ou la revendication 2, dans lequel chaque électrode (10) se présente sous la forme d'une aiguille hypodermique formée de platine et complètement isolée sur la totalité de son corps, seule l'extrémité étant sous tension.
